(19)

Europäisches
Patentamt
European
Patent Office
Office européen
des brevets

(11)  **EP 3 771 405 A1**

(12)  **EUROPEAN PATENT APPLICATION**

(43) Date of publication:
03.02.2021   Bulletin 2021/05

(51) Int Cl.:
*A61B 5/055* (2006.01)          *A61B 6/02* (2006.01)
*G16H 30/00* (2018.01)          *G16H 50/20* (2018.01)
*A61B 5/00* (2006.01)           *G01R 33/54* (2006.01)

(21) Application number: 20472008.0

(22) Date of filing: 20.07.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 02.08.2019   US 201962882076 P

(71) Applicant: **Smart Soft Ltd.**
**9000 Varna (BG)**

(72) Inventor: **Georgiev, Nedelcho Todorov**
**9000 Varna (BG)**

(74) Representative: **Georgieva-Tabakova, Milena**
**Lubenova**
**Tabakov,Tabakova & Partners**
**Law Firm**
**18, "Ami Bue" Street, entr. V**
**1606 Sofia (BG)**

(54)  **METHOD AND SYSTEM FOR AUTOMATED DYNAMIC MEDICAL IMAGE ACQUISITION**

(57)   A system and an automated method for medical image acquisition using dynamic workflow based on image processing, and more particularly automatic or semi-automatic diagnosis for medical conditions. Real-time identification of possible medical conditions helps recognize the areas of interest during the scan or MRI acquisition, which reduces the acquisition time and results in higher resolution images in areas where necessary at the expense of the total number of images.

EP 3 771 405 A1

## Description

### FIELD OF THE INVENTION

The present invention generally relates to a method and system for medical image acquisition using dynamic workflow based on image processing, and more particularly automatic or semi-automatic diagnosis for medical conditions such as herniated lumbar discs based on automatic reading of medical images and optionally additional patient information.

### BACKGROUND

In the domain of medical image analysis, diagnosis and recommendations are provided based on the results of medical images such as MRI (magnetic resonance imaging), CT scan (computerized tomography scan), X-ray and others.

Diagnosing medical conditions such as disc herniation is a time-consuming and demanding task which physicians have to perform manually. Since medical images such as MRI images comprise multiple layers, it is time-consuming for the physicians to check each layer for signs of possible disc herniation. The acquisition process takes a significant amount of time, and it is uncomfortable for the patient. Reducing that time can be achieved either by making a smaller number of scans or spending less time on each scan which would result in lower image resolution.

Magnetic resonance imaging (MRI), or nuclear magnetic resonance imaging (NMRI), is a technique of medical imaging used in radiology to produce a three-dimensional visual representation of the subject's anatomy. The MRI technology makes use of magnets that produce a strong magnetic field around the patient. The patient is placed lying in a large magnet and needs to stay still during the scanning process so as not to blur the image. The magnetic field causes the protons located in the tissue water of the patient to align with the magnetic field. A radiofrequency current is applied through the patient, the protons are stimulated and lose equilibrium. When the radiofrequency current is turned off, the protons realign with the magnetic field and some energy is released. The MRI scanning device detects that energy. Depending on the matter and the environment, the amount of energy and the time needed for the protons to realign with the field can vary. Relying on these properties, physicians can identify different types of tissues. The MRI scanning device sends the information to a computer, which in turn creates an image of the inner body, constructed using the information received from the scanning device. Contrast agents can be applied to the patient intravenously before or during the scanning process to increase the speed of proton realignment with the magnetic field. The produced image has brightness that is proportional to that speed.

An MRI system typically consists of a computer, a keyboard, a display, a radio frequency transmitter and a radio frequency receiver, gradient power supply, a radio frequency coil, a magnetic coil, and gradient coils. First, there is acquisition of the RF signals coming from the body of the patient. The process comprises multiple imaging cycles. During each of them, a series of RF pulses is directed at the body, then the gradients are activated and the signals are collected. A single imaging cycle cannot produce enough signal data for creating an image. This necessitates the use of many repetitions of the cycle. The duration of each cycle determines the time that is needed to acquire images. The protocols which are stored in the computer manage the acquisition. Different procedures have different corresponding protocols, so the operator can choose among them, and also alter settings for specific purposes. The data of the RF signal which is acquired is not initially represented as an image. The computer can create an image from that data using a Fourier transformation. The process is performed quickly, without slowing down the total time required for imaging. The images formed by the computer are also stored in it. The supported number of images is limited by the storage capacity of the computer. The process of displaying the images is controlled by the computer. It includes allowing the operator to select images and modify settings such as contrast and zoom level. Sometimes the user might need to have the characteristics of the images changed. The functions for altering those characteristics are carried out on the MRI system computer or another computer which is not part of the system.

A CT (computed tomography) scan is a procedure for producing medical images which involves X-ray measurements performed from various angles to create cross-sectional (tomographic) images of certain parts of an object. A CT system typically consists of a computer, gantry and table, operator's console and a monitor. The gantry is a round-shaped device which accommodates an X-ray tube, a data acquisition system and a detector array. The table is a device connected to the computer and the gantry. It moves according to the scanning program with high precision. The operator controls the scanner using the console. The console may comprise a keyboard, a mouse, a monitor and other devices. The computer serves as the connection between the operator and the rest of the imaging system components. It servers the following functions: controlling data acquisition, reconstructing images, storing the images and displaying them. Before initiating the data acquisition, the operator sets various scanning parameters. During the acquisition, the computer generates a series of X-rays, turning the detectors on and off at proper moments. It also transfers data and monitors the system state. To reconstruct the CT image, a lot of mathematical operations are performed, digitizing and reconstructing the data. The process is carried out by an array processor which serves as a specialized computer that performs calculations quickly and efficiently. The information acquired from the scan is recorded in a digital representation by the computer and the image is reconstructed. The software which executes the image reconstruction procedure is referred to as a reconstruction algorithm. The processing may affect the quality and the resolution. The algorithm is a part of the computer program and cannot be modified. Different algorithms are used for processing the data. Each of them is specific for the given equipment. The reconstructed images are acquired from the remaining radiation by one of the algorithms. The transmitted X-ray photons represent some amount of attenuation in the patient's body. They are compared with the intensity of the radiation emitted from the X-ray tube. The result of that comparison is relative transmission values. The attenuation values of various tissues are related to the attenuation value of water. These values can be arranged as a scale. They are called EMI (or Hounsfield) numbers and represent the CT digital numbers which are used to reconstruct the image. When the image is produced, the CT digital numbers correspond to a given brightness level. It can be modified to visualize various objects in the image. This operation is known as windowing. Modifying the width of the window results in modifying the contrast of the image. While an image is displayed, these values can be changed by the operator to enhance particular objects on the image. Finally, a monitor displays the reconstructed images. The operator can choose to view and examine specific images by changing the zoom level, brightness, contrast etc. For long-term storage of images, a separate system may be needed since the computer in the CT system has limited capacity. The image data can be transferred to another device such as a hard drive or an optical disc.

There are two parameters related to resolution in MRI images - basic resolution and phase resolution. Basic resolution is essentially the number of pixels in the readout direction, while phase resolution represents how many pixels there are in the phase direction.

There is a trade-off between resolution and signal-to-noise ratio (SNR). In MRI images, the resolution is determined by the number of pixels in a specified field of view. With higher resolution, small pathologies can be diagnosed more easily. However, increasing the basic resolution above a certain threshold results in the reduction of pixel size, thus reducing the signal-to-noise ratio and producing grains in the image. Higher resolutions can be achieved at the expense of prolonged acquisition time. On the other side, reducing the basic resolution below the acceptable range increases the signal-to-noise ratio and produces blurry images. Decreasing the phase resolution will increase the pixel size in one direction and change the horizontal-to-vertical proportion of the pixels.

Manipulating a grainy image is performed first by reducing the basic resolution, therefore reducing the acquisition time. Increasing the slice thickness leads to an increase in the voxel size and makes the image smoother. The field of view must also be increased, so as to increase the pixel size and smoothen the image even further. Manipulating a blurred image is performed first by increasing the basic resolution, therefore increasing the acquisition time. The field of view must be decreased, which results in the reduction of pixel size and signal-to-noise ratio, therefore making the image sharper. In essence, blurring is enhancing the image by reducing the noise, while sharpening shows edges more prominently.

The dependence between SNR and the other parameters can be given in the following form:

$$SNR \propto voxel\ volume \times \sqrt{acquisition\ time}$$

The voxel volume is derived from the dimensions of the pixel ($FOV_X/N_X$ and $FOV_Y/N_Y$), where: $FOV_X$ is the field of view in the phase-encoding direction, $FOV_Y$ is the field of view in the frequency-encoding direction, $N_X$ and $N_Y$ are the number of points in their respective matrices. The voxel volume can be further expressed as:

$$voxel\ volume = \frac{FOV_X}{N_x} \times \frac{FOV_Y}{N_y} \times slice\ thickness$$

The acquisition time depends on the number of frequency-encoding points and the time spent on each sample. Improvement of the SNR can be achieved by sampling the image several times, with all samples being averaged together. If RBW is the receiver bandwidth and $N_{avg}$ is the number of averaged samples, then the amount of time spent acquiring data can be expressed as:

$$acquisition\ time = \frac{N_X \times N_Y \times N_{avg}}{RBW}$$

A more detailed expression shows the full dependence between the acquisition parameters:

$$SNR \propto \frac{FOV_X}{N_x} \times \frac{FOV_Y}{N_y} \times \text{slice thickness} \times \sqrt{\frac{N_X N_Y N_{avg}}{RBW}}$$

Existing methods and systems designed for the automation of diagnosis have provided aid in the field of medical image analysis. Some of them have integrated artificial neural networks into the algorithm, but the limited number of medical images on which to train has hindered the achievement of high accuracy.

Moreover, MRI images comprise multiple layers - slices across the sagittal and axial plane. There are various acquisition techniques, the most common of which are T1 and T2. The contrast of TI-weighted MRI images depends on the longitudinal relaxation time of the tissue, while the contrast of T2-weighted MRI images depends on the transverse relaxation time. The difference between images made with different image acquisition devices is yet another obstacle - each device having its characteristics results in images with inherent differences. Therefore, the unification of all images from the same class remains a difficult task.

Neural networks have been around for a long time but only recently have they become widely used due to the increasing computing power of modern technology and the availability of graphics processing units (GPUs) - electronic circuits specialized in parallel computations and manipulation of matrices. They have proven to be very useful in the domain of neural networks since their operation involves multiplication of such structures. A neural network comprises multiple layers represented by matrices of coefficients, or so-called weights. The input passes through the network being subject to multiplication and addition which produce the output. If there is a certain number of possible outputs, also known as classes, the output is the probability measured in percentage for each of those classes to be the correct prediction.

It is a well-known fact that heterogeneous data is more difficult to deal with than homogenous data. The data may be diverse in terms of data type, format, data model, and semantics. There exist two main types of heterogeneity - syntactic and semantic heterogeneity. Syntactic heterogeneity is related to differences in data types, file formats, data encoding, data model, etc. In order to train a neural network, these differences need to be minimized. Therefore, pre-processing is often necessary to prepare the training set. The different sources of data mean that there exist some differences among the set, which in turn makes the learning process much more difficult due to the inability of such algorithms to recognize various representations of the same class. Semantic heterogeneity is related to differences in meanings and interpretations. Similarly to syntactic differences, semantic ones present the same difficulties to machine learning algorithms. In statistics, heterogeneity also refers to differences in statistical properties among the different parts of a dataset. This type of heterogeneity shows that statistical properties are not always similar across a complete dataset.

The human spine is composed of a chain of bones called the vertebrae. The front part of each vertebra is called the vertebral body. Between the vertebrae lie soft spinal discs. The discs have an elliptic shape and hard outer surface with a gel-like center (nucleus pulposus), the latter of which has variable thickness adjusting to pressure. The flexibility of the spine is due to the presence of those discs. The discs also absorb shocks that are directed at the spine during dynamic physical exercise such as running or jumping. Each disc resembles a balloon full of the gel-like substance which can start leaking out while under pressure. That condition leads to irritation on the nerves against the vertebrae, resulting in full or partial paralysis of the limbs. The medical condition called herniated disc, also known as slipped disc, means that one or more of the discs are protruding over the edges of the vertebral bodies.

Segmentation and evaluation of the herniated part on axial planes have not been implemented before, despite existing segmentation of the whole lumbar disc and the vertebrae. The means of achieving these objectives include the use of machine learning and more specifically convolutional neural networks.

One primary shortcoming of the methods for performing diagnostic procedures by means of computer technology is that they have so far relied solely on the images obtained by medical scanners and other image acquisition devices. No additional information has been used as input to a neural network along with the medical images. Thus, there remains the necessity to increase the recognition accuracy of medical conditions and automate the process of the diagnosis.

**BRIEF SUMMARY OF THE INVENTION**

The present invention, similarly to other existing methods and systems, facilitates the process of image acquisition, e.g., during MRI scans. A dynamic process of image acquisition, wherein later stages depend on the images that were received in the initial stages was suggested before, but our invention has a number of differences: The algorithm provides more accuracy and confidence of important findings in addition to speed improvements. The algorithm is based on pathology findings. Sometimes the plane and the field of view are not subject to change, with only the number of samples being altered. The process may be completely automated. Further speed improvements are achieved by the fact that classification of some images may be performed simultaneously while the acquisition of other images is in progress.

During a medical scan or MRI acquisition, the method can be used in the medical scanner or MRI equipment so as to identify the image areas of interest, to reduce acquisition time and produce higher resolution images where necessary. Irrelevant areas can be automatically disregarded, and more attention can be paid to the areas in question.

A method of dynamic image acquisition in which later stages depend on the images that were received in the initial stages is provided, the method providing higher accuracy and confidence of significant findings in addition to speed improvements.

**BRIEF DESCRIPTION OF THE DRAWINGS**

Exemplary embodiments of the disclosure are described with respect to the following drawings.

Figure 1 - FIG. 1 shows a block diagram of some of the components which may be utilized to perform a method for automated medical diagnostics and recommendations according to various embodiments described herein;

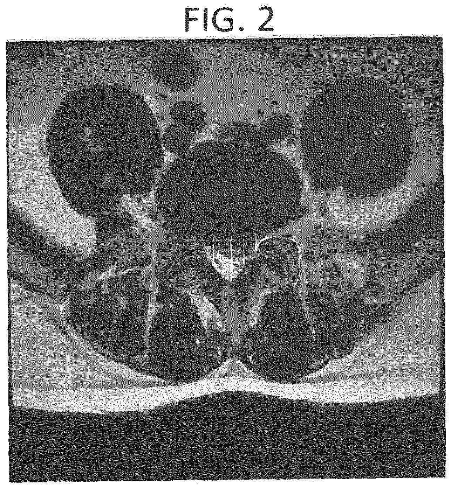

Figure 2 - FIG. 2 shows an example of segmentation and classification of the herniation according to its relative size and position according to various embodiments described herein;

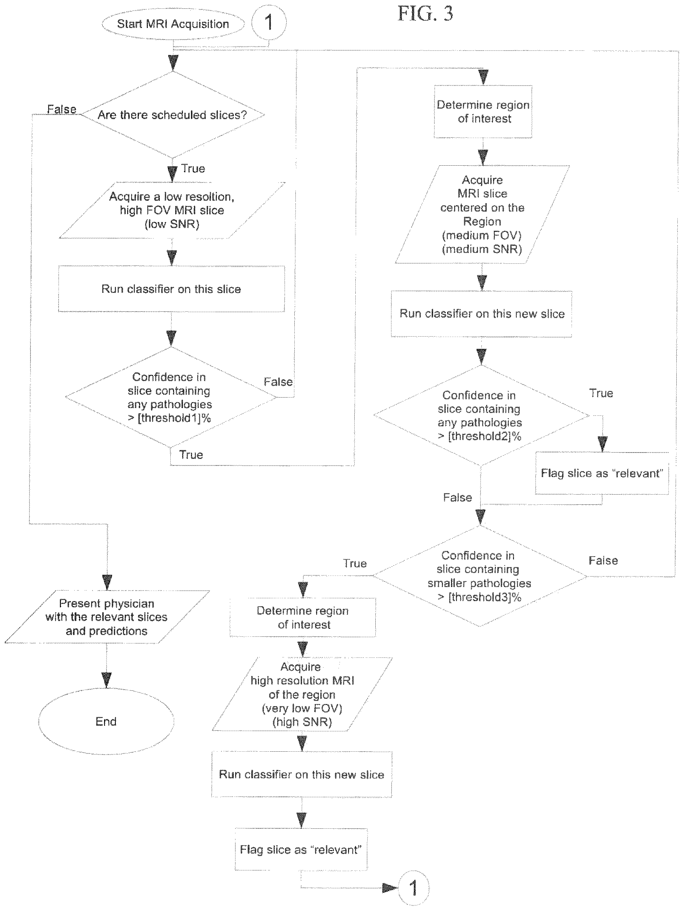

Figure 3 - FIG. 3 shows a flowchart of an example algorithm for dynamic automatic medical image acquisition according to various embodiments described herein;

**DETAILED DESCRIPTION OF THE INVENTION**

The present disclosure is to be considered as an exemplification of the invention, and is not intended to limit the invention to the specific embodiments illustrated by the figures or description below.

The input to the algorithm (for pathology classification) may comprise not only the given medical image but also additional patient information. It may be in the form of a questionnaire or other clinical data, the greater part of which can be subject to a numerical representation. To surpass the current state of the art, an algorithm has to cope with the heterogeneity of the data.

The methods of the invention may include a questionnaire part which can comprise several closed questions which aim to build some form of clinical history. The questions may be divided into the following groups: age, sex, body mass index; comorbidities (other known diseases and/or previous operations); trauma; timespan; pain; sensation; neurological deficit (abnormal function of a body area); held therapy; all of which relating to the medical condition.

The results produced by a system that uses the full information (clinical data and images) may predictively categorize the patient as negative, possible, suspicious, highly suggestive or definite of having a medical condition, such as a lumbar disc herniation.

The algorithm may be separated into three stages, and it may follow these steps: First, image feature extraction may be performed. It may include segmentation on the image to identify the areas of interest. The second stage may be categorization and evaluating a set of anatomical or pathological measurements. The third part of the neural network, produces diagnosis. The processing of two different domains of data combined means they have to be transformed into a single vector of a fixed number of dimensions. That may be achieved by creating two vectors with fixed dimensions representations, and after that concatenating them. For questions with numeric answers, such as 1/2/3 months, those numbers are plain quantities, and they may directly be passed as features to the neural network. However, not all questions included are suitable for this kind of representation. When the answers are not semantically gradual, the so-called one-hot encoding may be used. For example, five different answers are encoded with (0,0,0,0,1), (0,0,0,1,0), (0,0,1,0,0), (0,1,0,0,0), (1,0,0,0,0).

Work in real time may be supported during acquisition on the medical device. Usually, while scanning the patient, the physician needs to correctly position the medical acquisition device so that the proper scanned area is visible in the image. This process may be aided by the automated identification of the most important planes and areas of each image from images that are produced with shorter acquisition time. Once those are identified, the system performing the algorithm may choose the exact locations that need further examination and scan them with higher resolution or more accurate field of view, or greater signal to noise ratio, bigger number of samples or any number of them. The time that would be spent scanning irrelevant locations may be substituted with increasing the resolution of the ones that matter. The smaller number of slices means that for the same time spent during the scan or MRI acquisition, more quality images can be made without sacrificing information. The algorithm may also work in asynchronous mode while classification of other image slices is being performed. The quality of an image is considered better if it has higher resolution, or higher resolution with equal or better SNR, or better SNR with the same resolution.

In some embodiments, the algorithm initially gathers data by localizers - low-resolution images with a large field of view - and automatically detects the boundaries of the important areas. The algorithm plans the axial slices using the sagittal and coronal localizers, and the sagittal slices using the coronal and axial localizers. In other embodiments, these areas may be predefined.

The planned images or image blocks start to be acquired. These initially planned images are generally obtained in a mode that is quicker than the mode used in a standard process. This process continues until there are no more scheduled image acquisitions. When the first images are completely acquired, classification starts for them and later for all others that are gathered. Probabilities for different pathologies are provided by algorithms. If the probabilities for a specific pathology are over a certain threshold, new additional acquisitions are scheduled. In some embodiments, the region of interest is automatically detected. The region of interest is the region which contains the tissues affected by pathology. The new additional acquisitions in further embodiments may be a new number of samples of the same slide. In other embodiments, they may represent additional slices with a smaller distance between slices or slices with higher resolution or more accurate field of view, or greater signal-to-noise ratio, or any number of these. In further embodiments, the slides that were not originally scheduled are also subject to classification and segmentation in order to schedule a third group of acquisitions with even higher resolution. In still other embodiments, there is no third group of acquisitions.

When there are no more scheduled image acquisitions, all images/slides from the initial plan and those that are additionally gathered may be combined in the right space order, and those from the same plane may be combined preserving the spatial correspondence in a "picture-in-picture" fashion, producing an image with varying quality - higher in relevant areas in the additional images, lower in less relevant areas. Other ways of combining the images/slides from the initial plan with the ones additionally gathered are by creating an image from the average pixel values of the former and the latter, or creating additional slices in new planes.

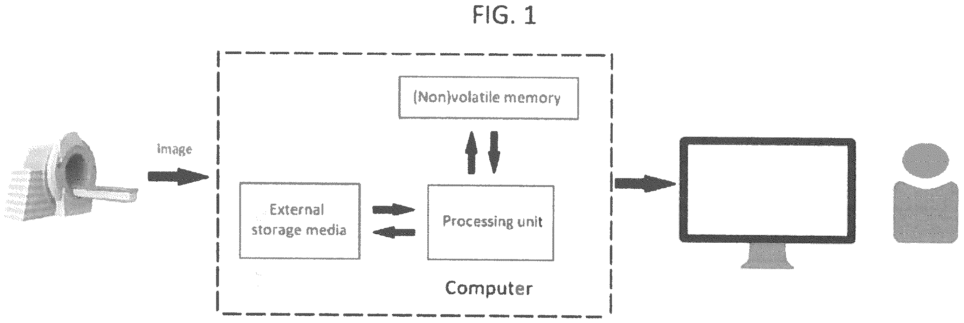

FIG. 1 illustrates an example of some physical components of a system for automated medical diagnostics which may be used to complete one or more of steps of the methods described herein. In some embodiments, one or more steps may be implemented using a general purpose desktop computer, laptop or a microprocessor which has been programmed in accordance with the algorithm described herein. In other embodiments, one or more steps may be implemented via a processing system which may include some or all of the following: a motherboard, a CPU (central processing unit), volatile and/or non-volatile memory (e.g., RAM, DRAM, SRAM, SDRAM, ROM, PROM, EPROM, EEPROM, NVRAM, Flash RAM), external storage media (e.g., a hard disk, CD-ROM etc.) and other optional special purpose logic devices (e.g., ASICS), or configurable ones (e.g., reprogrammable FPGA).

The system may also include a set of input devices, such as a keyboard and mouse, and a display controller that controls the output to a monitor which serves as visual interface between the user and the processing system and it displays a graphical representation of the medical image which is modified to show where the pathology is located. A network interface may also be required for communication via a network, such as the Internet or an intranet. In such settings, communication between with an imaging device or an image database may be performed via the network, or via an input/output interface. Computer readable devices, such as hard disks, compact discs or flash drives, are required in order to store the executable instructions.

**Claims**

1. A method of dynamic medical image acquisition by identification in real time of pathologies, the method comprising:

   acquiring a high-FOV slice with low acquisition time;
   running a classifier on the high-FOV slice with low acquisition time;
   if the predicted probability of the high-FOV slice with low acquisition time containing any pathologies, according to the run classifier, is higher than a set threshold, acquiring one or more second-type slices within the region of the high-FOV slice with better quality;
   combining the acquired high-FOV slices and second-type slices, according to their spatial order into an image with higher quality in the areas covered by slices with better quality, the combining performed by replacing the high-FOV slices with the second-type slices from the same plane.

2. The method of claim 1, wherein the step between acquiring a second-type slice and combining the acquired slices comprises:

   running a classifier on the second-type slice;
   for each second-type slice, if the predicted probability of the second-type slice containing any pathologies, according to the run classifier, is higher than a set threshold, acquiring one or more better-quality slices within the region of the second-type slice, until there are no more slices with probability of containing any pathologies, according to the run classifier, that probability being higher than the set threshold;
   flagging the second-type slice as relevant if the probability of it containing any pathologies, according to the run classifier, is higher than a set threshold;

marking a region which contains tissues affected by pathology as a region of interest if the probability, according to the run classifier, of the flagged slice containing pathologies detectable only on higher-resolution images, is higher than a set threshold;

acquiring a high-resolution low-FOV slice of the region of interest.

3. The method of claim 1, wherein the characteristics of the stages relating to the second-type slices are determined simultaneously with acquiring the high-FOV slices.

4. The method of claim 1, wherein during the stages relating to the second-type slices, images from new planes are acquired, those planes being selected by the algorithm if the algorithm determines that using an acquisition slice from those planes would yield more findings.

5. The method of claim 1, wherein the combining of high-FOV slices and second-type slices is performed in one of the following ways: creating an image from the average pixel values of the former and the latter; creating additional slices in new planes; merging the former and the latter in a picture-in-picture fashion.

6. A system for dynamic medical image acquisition with identification in real time of pathologies, configured to:

acquire a high-FOV slice with low acquisition time;

run a classifier on the high-FOV slice with low acquisition time;

if the predicted probability of the high-FOV slice with low acquisition time containing any pathologies, according to the run classifier, is higher than a set threshold, acquire one or more second-type slices within the region of the high-FOV slice with better quality;

combine the acquired high-FOV slices and second-type slices, according to their spatial order into an image with higher quality in the areas covered by slices with better quality, the combining performed by replacing the high-FOV slices with the second-type slices from the same plane.

7. The system of claim 6, wherein the system is configured to perform the step between acquiring a second-type slice and combining the acquired slices by:

running a classifier on the second-type slice;

for each second-type slice, if the predicted probability of the second-type slice containing any pathologies, according to the run classifier, is higher than a set threshold, acquiring one or more better-quality slices within the region of the second-type slice, until there are no more slices with probability of containing any pathologies, according to the run classifier, that probability being higher than the set threshold;

flagging the second-type slice as relevant if the probability of it containing any pathologies, according to the run classifier, is higher than a set threshold;

marking a region which contains tissues affected by pathology as a region of interest if the probability, according to the run classifier, of the flagged slice containing pathologies detectable only on higher-resolution images, is higher than a set threshold;

acquiring a high-resolution low-FOV slice of the region of interest.

8. The system of claim 6, wherein the system is configured to determine the characteristics of the stages relating to the second-type slices simultaneously with acquiring the high-FOV slices.

9. The system of claim 6, wherein the system is configured to acquire images from new planes during the stages relating to the second-type slices, those planes being selected by the algorithm if the algorithm determines that using an acquisition slice from those planes would yield more findings.

10. The system of claim 6, wherein the system is configured combine the high-FOV slices and second-type slices in one of the following ways: creating an image from the average pixel values of the former and the latter; creating additional slices in new planes; merging the former and the latter in a picture-in-picture fashion.

11. A computer program product comprising computer-readable instructions which, when loaded and executed on a suitable system, perform the steps of a method according to claims 1-5.

FIG. 1

Non-volatile memory

Processing unit

External storage media

Computer

Image

FIG. 2

FIG. 3

Start MRI Acquisition ①

Are there scheduled slices?

False

True

Acquire a low resoltion, high FOV MRI slice (low SNR)

Run classifier on this slice

Confidence in slice containing any pathologies > [threshold1]%

False

True

Present physician with the relevant slices and predictions

End

Determine region of interest

Acquire MRI slice centered on the Region (medium FOV) (medium SNR)

Run classifier on this new slice

Confidence in slice containing any pathologies > [threshold2]%

True

Flag slice as "relevant"

False

Confidence in slice containing smaller pathologies > [threshold3]%

True

False

Determine region of interest

Acquire high resolution MRI of the region (very low FOV) (high SNR)

Run classifier on this new slice

Flag slice as "relevant"

①

Europäisches
Patentamt

European
Patent Office

Office européen
des brevets

## EUROPEAN SEARCH REPORT

**Application Number**

EP 20 47 2008

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/210053 A1 (PIRON CAMERON A [CA] ET AL) 26 July 2018 (2018-07-26)<br>* paragraphs [0004], [0012], [0014] – [0020], [0024] *<br>* figures 1-2 *<br>----- | 1-11 | INV.<br>A61B5/055<br>A61B6/02<br>G16H30/00<br>G16H50/20<br>A61B5/00 |
| A | RU 2 654 199 C1 (SAMSUNG ELECTRONICS CO LTD [KR]) 16 May 2018 (2018-05-16)<br>* the whole document *<br>----- | 1-11 | G01R33/54 |
| A | WO 2009/050676 A1 (KONINKL PHILIPS ELECTRONICS NV [NL] ET AL.)<br>23 April 2009 (2009-04-23)<br>* the whole document *<br>----- | 1-11 | |

**TECHNICAL FIELDS SEARCHED (IPC)**

A61B
G16H
G01R
G06T

The present search report has been drawn up for all claims

| Place of search | Date of completion of the search | Examiner |
|---|---|---|
| The Hague | 26 November 2020 | Faymann, Juan |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
### ON EUROPEAN PATENT APPLICATION NO.

EP 20 47 2008

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2020

| Patent document cited in search report | Publication date | Patent family member(s) | Publication date |
|---|---|---|---|
| US 2018210053 A1 | 26-07-2018 | US 2018210053 A1<br>WO 2017009691 A1 | 26-07-2018<br>19-01-2017 |
| RU 2654199 C1 | 16-05-2018 | NONE | |
| WO 2009050676 A1 | 23-04-2009 | NONE | |

EPO FORM P0459

For more details about this annex : see Official Journal of the European Patent Office, No. 12/82